Europäisches Patentamt

European Patent Office

Office européen des brevets

(11) Publication number: **0 279 661**
A2

(12)
# EUROPEAN PATENT APPLICATION

(21) Application number: 88301348.4

(22) Date of filing: 18.02.88

(51) Int. Cl.⁴: **C 12 N 15/00**
C 12 P 21/00, C 12 N 7/00,
C 07 K 15/04, A 61 K 39/15

(30) Priority: 19.02.87 GB 8703864
24.04.87 GB 8709779

(43) Date of publication of application:
24.08.88 Builetin 88/34

(84) Designated Contracting States:
AT BE CH DE ES FR GB GR IT LI LU NL SE

(71) Applicant: OXFORD VIROLOGY LIMITED
10 Storey's Gate
Westminster London SW1P 3AY (GB)

Bishop, David H.L.
15 Rawlinson Road
Oxford OX2 6UE (GB)

(72) Inventor: Bishop, David H. L.
15 Rawlinson Road
Oxford, OX2 6UE (GB)

Roy, Polly
15 Rawlinson Road
Oxford, OX2 6UE (GB)

(74) Representative: Ritter, Stephen David et al
Mathys & Squire 10 Fleet Street
London EC4Y 1AY (GB)

(54) Production of bluetongue virus antigens using a baculovirus expression vector.

(57) Bluetongue virus proteins are produced in an insect/baculovirus expression system. Particularly, DNA sequences corresponding to the gene that codes for the BTV neutralization antigen VP2 and for the group-specific antigen VP3 have been inserted into a baculovirus transfer vector in lieu of the 5′ coding region of the polyhedrin gene of Autographa californica nuclear polyhedrosis virus (AcNPV). After cotransfection of Spodoptera frugiperda cells with wild-type AcNPV DNA in the presence of the derived recombinant transfer vector DNA, polyhedrin-negative recombinant baculoviruses were recovered. S. frugiperda cells infected with these recombinant viruses, produce proteins similar in size and antigenic properties to the BTV VP2 and VP3 proteins were synthesized.

EP 0 279 661 A2

## Description

PRODUCTION OF BLUETONGUE VIRUS ANTIGENS USING A BACULOVIRUS EXPRESSION VECTOR

This invention relates to a process for producing bluetongue virus proteins, particularly antigens VP2 and VP3.

BTV is the prototype virus of the Orbivirus genus (Reoviridae family). The virus contains a segmented genome consisting of 10 double-stranded (ds) RNA molecules each of which is unique and is believed to code for a single polypeptide product (Gorman et al., 1981; Sangar and Mertens, 1983).

This ten segment, double-stranded RNA genome of the virus is located in the core of the BTV particle, a core which also contains 2 major (VP3 and VP7) and 3 minor protein species (VP1, VP4, VP6). The core is surrounded by an outer capsid consisting of 2 major proteins, VP2 and VP5 to give a complete virion particle with a diameter of approx. 69 nm (Martin and Zweerink, 1972; Huismans, 1979; Mertens et al., 1984). The outer capsid protein, VP2, had been implicated as responsible for the elicitation of neutralizing antibody while VP3 with VP7 can elicit group-reactive antibody (Huismans and Erasmus, 1981; Huismans et al., 1979).

It has been demonstrated both in vivo (using intertypic reassortant viruses, Kahlon et al., 1983) and in vitro (by RNA translation, Sangar and Mertens, 1983) that BTV RNA segment 2 codes for VP2. Immunoprecipitation techniques (Huismans and Erasmus (1981)) have shown that VP2 is a major serotype-specific antigen. Also it has been demonstrated that solubilized VP2 polypeptide induces neutralizing antibodies that protect sheep against viral infection (Huismans et al., 1983).

In order to analyse the structure of the VP2 gene, we have previously synthesized a DNA copy of RNA segment 2 of BTV serotype 10. The DNA was inserted into the plasmid pBR322 at the PstI site using dC-dG tailing and the complete nucleotide sequence of the DNA (2926 base pairs) was determined from overlapping clones (Purdy et al., 1985).

We have recently published the complete nucleic acid sequence of RNA segment 3 (L3) from BTV serotypes 10 and 17 (Purdy et al., 1984; Ghiasi et al., 1985). Comparison of these sequences showed them to be highly conserved both at the nucleic acid and amino acid levels. The primary gene product (VP3) of these genes differed by only 9 amino acids. Using the DNA of BTV-17 L3, we have demonstrated hybridization of this probe with the equivalent segments of the 19 BTV serotypes isolated from different geographical locations.

In our previously published European Patent Application No 0 178 435 there is described the production of cDNA probes for detection of bluetongue virus RNA. While such probes are of considerable utility, for example in the detection of RNA-containing infectious material in carrying out diagnostic tests, there is a need to be able to produce bluetongue virus proteins (and polypeptides comprising at least an antigenic portion of such proteins) by recombinant DNA technology.

Previous attempts to achieve this end have, however, proved to be unsuccessful. Thus a number of groups have attempted, but failed, to achieve expression of bluetongue proteins in expression systems based on Vaccinia. Furthermore there have been no reports of successful attempts to express bluetongue virus proteins in conventional expression systems, e.g. those based on E. coli and other bacteria and yeasts. The failure of such methods is believed to be at least in part accounted for by the relatively large size of the bluetongue virus proteins, or by the fact that the promotors of conventional expression systems are ineffective for promoting the expression of bluetongue virus proteins.

We have now surprisingly found that expression systems based on insect cells can efficiently be used to achieve expression of antigenically competent bluetongue virus proteins.

Thus according to the present invention there is provided a process for producing a polypeptide comprising at least an antigenic portion of a bluetongue virus protein which comprises infecting susceptible insects or cultured insect cells with an expression vector having a DNA segment coding for said polypeptide. Examples of such polypeptides include the VP2 and VP3 proteins of bluetongue virus.

Especially suitable expression vectors are those based on baculoviruses. Thus for example the expression vectors used in the method of the invention may comprise a recombinant baculovirus having a DNA segment coding for a polypeptide comprising at least an antigenic portion of a bluetongue virus protein.

Such recombinant baculoviruses may include promotor systems native to naturally occurring baculoviruses, for example the so-called "polyhedrin" promotor or they may include other promotor systems capable of directing expression of polypeptide in transformed insect or cultured insect cells.

The invention also includes such recombinant baculoviruses themselves as well as protein produced by the process of the invention.

By the term "comprising at least an antigenic portion" as used herein to refer to a polypeptide is meant a polypeptide which is capable of exhibiting an antigenic property of a native bluetongue virus protein, e.g. the capability of binding to an antibody to a bluetongue virus protein.

Such a portion may be one which is incapable of exhibiting said antigenic property per se, but which can do so when linked to a suitable adjuvant. Normally the antigenic portion would be at least 5 and preferably at least 10 amino acids long. There is no critical upper limit on the size of such polypeptides and they may, for example, consist of polypeptides of a size comparable to native bluetongue virus proteins.

The expression and characterization of the BTV-10 VP2 gene product and the BTV-17 L3 gene product using an expression system based on recombinant baculoviruses is illustrated by the following Examples. In the case of VP2 the expressed protein has been shown to correspond in size to BTV induced VP2 protein, to react with BTV-10 antisera and, in mice or rabbits, induce antibodies that neutralize infections BTV-10 and to lesser extents BTV-11 and BTV-17, but not BTV-13.

Similarly, for the BTV-17 L3 gene product the expressed protein has been shown to correspond in size to BTV induced VP3 protein, to react with antisera to U.S. serotypes, BTV-10, 11, 13 and 17 and, in mice or rabbits, induce antibodies that react with BTV-10 or BTV-17 VP3 antigen.

In both Examples, AcNPV and recombinant virus stocks were grown and assayed in confluent monolayers of S. frugiperda cells in medium containing 10% fetal bovine serum according to the procedures described by Brown and Faulkner (1977).

## EXAMPLE 1 - Expression of VP2

### A. DNA manipulations and construction of DNA clones.

Plasmid DNA manipulations were carried out following the procedures described by Maniatis and associates (1982). Restriction enzymes, T4 DNA ligase and Klenow large fragment of DNA polymerase, were purchased from New England Biolabs Inc. (Beverly, Mass., USA). Calf intestine alkaline phosphatase was obtained from Boehringer Mannheim GmbH (FGR). Two BTV-10 segment 2 DNA clones representing nucleotides 1-1667 and 1471-2926 of the gene (Purdy et al., 1985) were used to construct a single copy of the entire coding region of the gene using a unique BssHII site present in the overlapping regions of the plasmids and the BamHI site of pBR322. After ligation and transformation, plasmids containing the entire coding sequence of the gene were identified and characterized by restriction enzyme analyses. One clone, pMP5, was employed for the subsequent transfer vector manipulations.

### B. Insertion of BTV-10 segment 2 DNA into a baculovirus transfer vector.

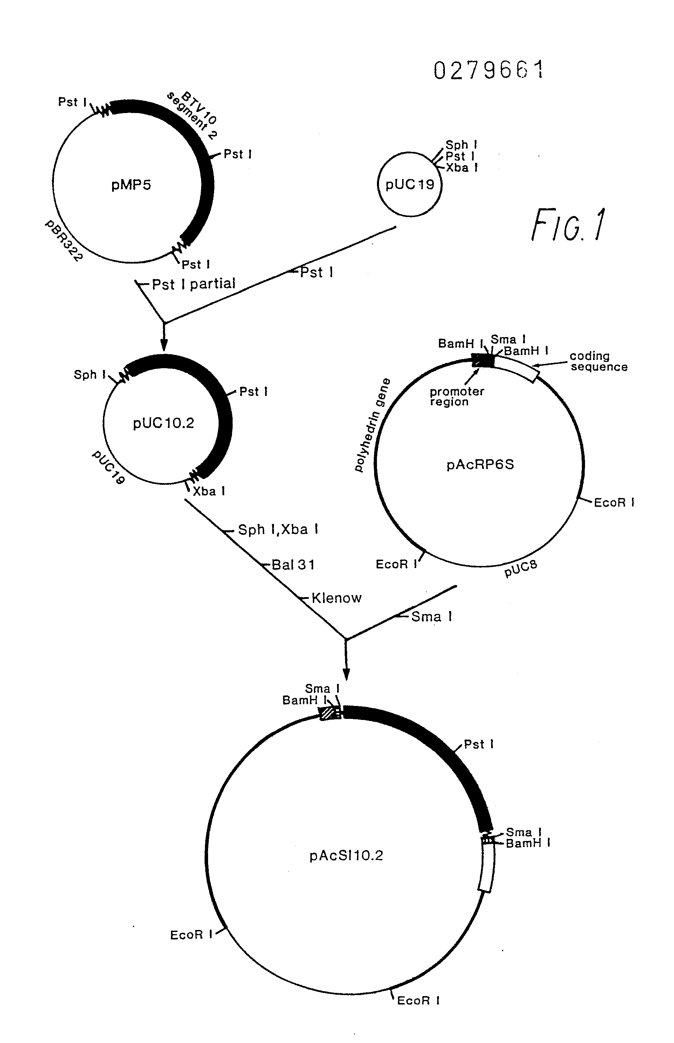

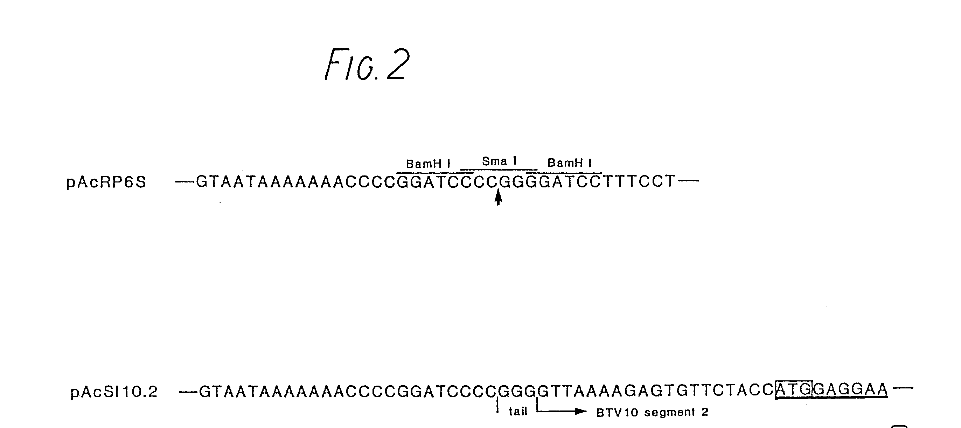

The unique BamHI vector plasmid derived from AcNPV (pAcRP6, Matsuura et al., 1986) was modified by the insertion of a BamHI-SmaI adapter to give a new vector designated pAcRP6S. Since BTV-10 segment 2 DNA has an internal PstI site (nucleotide 1727) the pMP5 plasmid DNA was partially digested with PstI and the 3 kb DNA containing the complete gene was recovered and cloned into the PstI site of plasmid pUC19. Derived plasmids containing the BTV VP2 gene were recovered, characterized by the appropriate restriction enzyme analyses and the viral DNA excised by digestion with SphI and XbaI, followed by Bal31 exonuclease digestion to eliminate the dC-dG terminal sequences. The product DNA was repaired with the Klenow fragment of DNA polymerase and ligated into the dephosphorylated SmaI site of the pAcRP6S transfer vector. Recombinant transfer vectors were recovered and characterized by restriction enzyme and sequence analyses (Maxam and Gilbert, 1980). Recombinant pAcSI10.2 was shown to lack the majority of the dC-dG tails and to have the BTV DNA in the right orientation for expression by the AcNPV polyhedrin promoter (Fig. 1, 2).

### C. Transfection and selection of recombinant viruses.

S. frugiperda cells were transfected with mixtures of purified infectious AcNPV DNA and pAcSI10.2 plasmid DNA by a modification of the procedures described by Smith and associates (1983). AcNPV DNA (1 μg), purified by the method of Smith and Summers (1978), was mixed with various concentrations of plasmid DNA (10-100 ug) and adjusted to 950 ul with Hepes buffered saline (20 mM Hepes buffer, 1 mM Na$_2$HPO$_4$, 5 mM KCl, 140 mM NaCl, 10 mM glucose, pH 7.05). After precipitation with 50 ul of 2.5 M CaCl$_2$, DNA was inoculated onto monolayers of 1.5 × 10$^6$ S. frugiperda cells in 35 mm tissue culture dishes and incubated for 1 hr at room temperature. The supernatant fluids were discarded and 1.5 ml of medium containing 10% fetal bovin serum were added. After 4 days of incubation at 28°, the supernatant fluids were harvested and titrated in confluent monolayers of S. frugiperda cells. Plaques exhibiting no evidence of occlusion bodies (viral polyhedra) as determined by transmission light microscopy were recovered and retitered on S. frugiperda cells to obtain combinant, polyhedrin-negative, viruses. Following a third plaque picking, high titered stocks (10$^{7-8}$ PFU/ml) of the recombinant viruses (RP6S2.1, RP6S2.2) were obtained using monolayer cultures of S. frugiperda cells.

### D. Extraction and characterization of viral and cellular nucleic acids.

To obtain recombinant virus DNA, S. frugiperda cells were infected with virus at a multiplicity of 10 PFU/cell and incubated at 28° for 48 hr. The infected cells were harvested, briefly sonicated to obtain cell lysates and centrifuged for 10 minutes at 500 × g to remove cell debris. The supernatant fluids were recovered. The procedures used for virus isolation and subsequent viral DNA extraction were essentially similar to those described by Matsuura et al. (1986). DNA was resuspended in water and stored at -20°. For Southern analyses, preparations of viral DNA were digested to completion with BamHI and the products resolved by electrophoresis in 0.8% agarose (BRL, Madison, WI) then blotted to "Genescreen Plus" (New England Nuclear Corp., NEN, Boston, Mass.). After drying, DNA was hybridized to nick-translated BTV 10 segment 2 DNA obtained from clone pAcSI10.2 (Southern, 1975). The membranes were washed and autoradiographed.

To obtain infected cell RNA, S. frugiperda cells were inoculated with virus at a multiciplicity of 10 PFU/cell and incubated for 36 hr at 28° in medium. RNA was extracted from the infected cells by the procedure of Hefti and Bishop (1975). Total infected cell RNA was chromotographed on columns of oligodeoxythymidylic acid-cellulose to separate RNA species containing polyadenylic acid sequences. The RNA preparations were treated with 10 mM methylmercury hydroxide (Bailey and Davidson, 1976), resolved by electrophoresis in 1% agarose containing 10 mM methylmercury and transferred by blotting in "Genescreen Plus" (Alwine et al.,

1977), the membranes were then dried by the method of Denhardt (1966). After hybridization the membranes were washed and autoradiographed.

E. Protein and immunoprecipitation analyses.

S. frugiperda cells were infected with recombinant virus (RP6S2.2) at a multiplicity of 10 PFU/cell in 35 mm tissue culture dishes and incubated at 28° for 48 hr. Each dish was then labelled with 100 μCi of [35S]methionine (NEN, 1154 ci/m mole) for 2 hr in methionine-free medium. BHK-21 cells were similarly infected with BTV-10 at a multiplicity of 10 PFU/cell and after 18 hr of incubation at 37°, the cells were labelled with 75 uCi of [35S]methionine (NEN, 1154 Ci/m mole) for 2 hr in methionine-free medium. Prior to labelling, the cells were incubated for 1 hr in methionine-free medium to reduce the intracellular pools of the precursor. After the labelling periods, the medium was removed, the monolayers rinsed three times with cold PBS and the cells lysed in 500 μl of RIPA buffer (1% Triton X-100, 1% sodium deoxycholate, 0.15 M NaCl, 0.05 M Tris-HCl, 0.01 M EDTA, 0.1% SDS, pH 7.4). Aliquots of 10 μl of the extracts were incubated with 5-10 μl of the appropriate antiserum for 1 hr at room temperature before addition of 25 ul of a suspension of 100 mg protein A-Sepharose CL-4B beads (Sigma, St. Louis, MO) in RIPA buffer. Following a further 1 hr incubation at room temperature, the beads were recovered by centrifugation, washed 3 times with cold RIPS buffer and the immune complexes that were bound to the beads were removed by boiling for 5 minutes in dissociation buffer (2.3% SDS, 10% glycerol, 5% 2-mercaptoethanol, 62.5 mM Tris-HCl, 0.01% bromophenol blue, pH 6.8) followed by centrifugation. Aliquots of the supernatant fluids were subjected to electrophoresis in 10% discontinuous gels of polyacrylamide as described in Laemmli (1970). After electrophoresis, the gels were impregnated with fixing solution (40% methanol, 10% acetic acid in water), dried and exposed at -70°C to X-ray film.

F. Production of antibodies in mice and rabbits.

S. frugiperda cells infected with the RP6S2.2 recombinant were collected and subjected to freezing and thawing, followed by low speed centrifugation. The supernatant materials were used to raise antibodies in mice or rabbits. For mice, each animal received one intrapertoneal injection of antigen in Freund's complete adjuvant on day 0, followed by three injections of antigen in Freund's incomplete adjuvant on days 7, 14 and 21 and one intrapertoneal injection of 2 × 10^6 Ehrlich's ascites cells on day 18. Ascitic fluids were removed at intervals from day 22 to day 34. For rabbits, each animal received one intramuscular injection of antigen in Freund's incomplete adjuvant on days 7 and 16. Blood was collected on day 14 and on day 22.

G. Neutralisation of BTV with mouse ascitic fluids or rabbit sera immunized with recombinant virus infected cell extracts.

Mouse ascitic fluids and rabbit sera were diluted as indicated with PBS. Seed virus was diluted as indicated with PBS containing 0.075% bovine serum albumin. Virus neutralization was accomplished by mixing 100 μl of each dilution of mouse ascitic fluids (or rabbit sera) with 100 μl of each virus dilution followed by 1 hr incubation at room temperature. After incubation, the samples were added to 0.5 ml of a suspension of Vero cells (3 × 10^5 cells/ml in Eagle's minimum essential medium supplemented by 2% fetal bovine serum) in Linbro plates with 24 flat bottom wells (Flow Laboratories, McLean Va) and incubated at 35° for 1 hr. The cells were overlaid with 0.75% carboxymethylcellulose in Eagle's minimum essential medium. After 3 days incubation at 35°, the monolayers were fixed with 10% (v/v) formalin in PBS for 10 min and stained with 1.5% crystal violet in ethanol. Plaques in each well were then counted.

H. Immunoprecipitation of proteins using mouse ascitic fluids or rabbits antisera.

The mouse ascitic fluids and rabbit sera were diluted 25 times with RIPA buffer and incubated with [35S]methionine labelled BTV-10 infected BHK-21 cell lysates for 1 hr prior to immunoprecipitation and gel analyses as described above.

EXAMPLE 2 - Expression of VP3

A. Insertion of BTV-17 segment 3 DNA into a baculovirus transfer vector.

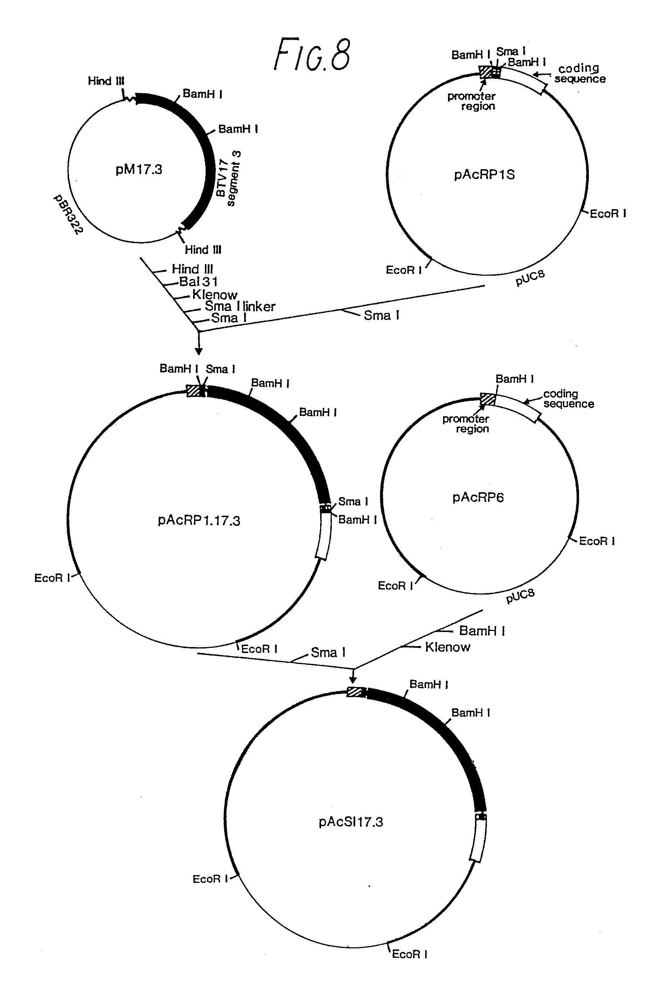

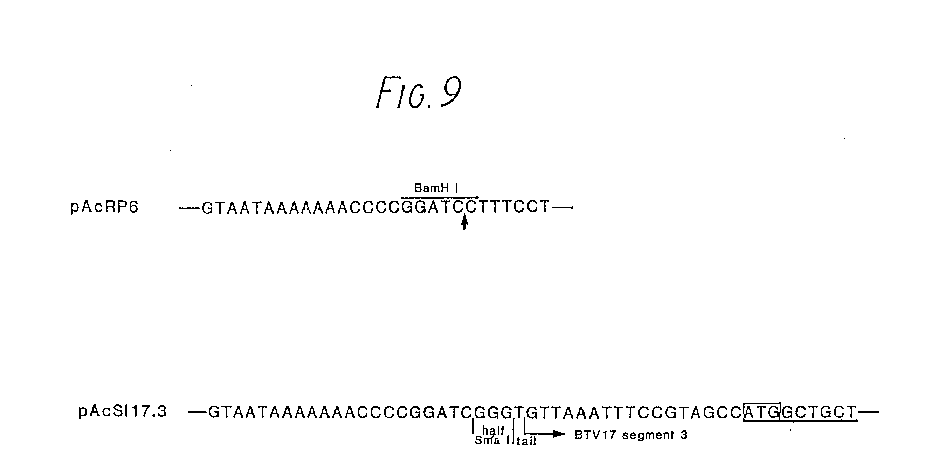

Plasmid DNA manipulations were carried out following the procedures described by Maniatis and Associates (1982). Restriction enzymes, T4 DNA ligase and the Klenow large fragment of DNA polymerase, were purchased from New England Biolabs Inc. (Beverly, Mass., USA). Calf intestine alkaline phosphatase was obtained from Boehringer Mannheim GmbH (FGR). BTV 17 segment 3 DNA was excised from pBR322 by digestion with Hindi III followed by Bal 31 exonuclease digestion to eliminate the dA-dT terminal sequences. The product DNA was repaired with the Klenow fragment of DNA polymerase and ligated into the Sma I linker. Following the digestion with Smal, the DNA was then ligated into the dephosphorylated unique Smal site of a baculovirus transfer vector plasmid derived from AcNPV (pAcRP1S, Possee et al., 1986). Since a new transfer vector (pAcRP6) has been modified with a unique BamH1 site (Matsuura et al., 1986), BTV-17 segment 3 DNA was excised from pAcRP1S transfer vector (pAcRP1.17.3) with Smal and religated into the dephosphorylated BamHI site of the modified transfer vector pAcRP6. Recombinant transfer vectors were recovered and characterized by restriction enzyme and sequence analyses (Maxam and Gilbert, 1980). Recombinant pAcSI17.3 was shown to consist of only one T residue of dA-dT tails and to have the BTV DNA in the right orientation for expression by the AcNPV polyhedrin promoter (Fig. 8, 9).

B. Transfection and selection of recombinant viruses.

S. frugiperda cells were transfected with mixtures of purified infectious AcNPV DNA and pAcSI17.3 plasmid DNA by a modification of the procedures described by Smith and Associates (1983). AcNPV DNA (1 µg), purified by the method of Smith and Summers (1978), was mixed with various concentrations of plasmid DNA (10-100 µg) and adjusted to 950 µl with Hepes buffered saline (20 mM Hepes buffer, 1 mM Na2HPO4, 5 mM KC1, 140 mM NaC1, 10 mM glucose, pH 7.05). After precipitation with 50 µl of 2.5 M CaCl2, DNA was inoculated onto monolayers of 1.5 × 10⁶ S. frugiperda cells in 35 mm tissue culture dishes and incubated for 1 hr at room temperature. The supernatant fluids were discarded and 1.5 ml of medium containing 10% fetal bovine serum were added. After 4 days of incubation at 28°, the supernatant fluids were harvested and titrated in confluent monolayers of S. frugiperda cells. Plaques exhibiting no evidence of occlusion bodies (viral polyhedra) as determined by transmission light microscopy were recovered and retitered on S. frugiperda cells to obtain recombinant, polyhedrin-negative, viruses. Following a third plaque picking, high titered stocks (10⁷⁻⁸ PFU/ml) of the recombinant virus (RP6S3.11) was obtained using monolayer cultures of S. frugiperda cells.

C. Southern blot hybridization.

To obtain recombinant virus DNA, S. frugiperda cells were infected with virus at a multiplicity of 10 PFU/cell and incubated at 28° for 48 hr. The infected cells were harvested, briefly sonicated to obtain cell lysates and centrifuged for 10 min at 500 × g to remove cell debris. The supernatant fluids were recovered. The procedures used for virus isolation and subsequent viral DNA extraction were essentially similar to those described by Matsuura et al., (1986). DNA was resuspended in water and stored at -20°. For Southern analyses, preparations of viral DNA were digested to completion with EcoRV and HindIII and the products resolved by electrophoresis in 0.8% agarose (BRL, Madison, WI) then blotted to "Genescreen Plus" (New England Nuclear Corp., NEN, Boston, Mass.) After drying, DNA was hybridized to nick-translated BTV 17 segment 3 DNA obtained from clone pAcSI17.3 (Southern, 1975). The membranes were washed and autoradiographed.

D. Northern blot hybridization.

To obtain infected cell RNA, S. frugiperda cells were inoculated with RP6S3.11 recombinant or with wild-type AcNPV virus at a multiplicity of 10 PFU/cell and incubated for 36 hr at 28° in medium; RNA was extracted from the infected cells by the procedure of Hefti and Bishop (1975). Total infected cell RNA was chromatographed on columns of oligodeoxythymidylic acid-cellulose to separate RNA species containing polyadenylic acid sequences. The RNA preparations were treated with 10 mM methylmercury hydroxide (Bailey and Davidson, 1976), resolved by electrophoresis in 1% agarose containing 10 mM methylmercury and transferred by blotting in "Genescreen Plus" (Alwine et al., 1977). The membranes were then dried, and hybridized to ³²P-labelled, nick-translation products of the appropriate viral DNA as described by Denhardt (1966). After hybridization the membranes were washed and autoradiographed.

E. Protein and immunoprecipitation analyses.

S. frugiperda cells were infected with recombinant virus (RP6S3.11) at a multiplicity of 10 PFU/cell in 35 mm tissue culture dishes and incubated at 28° for 48 hr. Each dish was then labelled with 100 uCi of [³⁵S]methionine (NEN, 1154 Ci/m mole) for 2 hr in methionine-free medium. BHK-21 cells were similarly infected with BTV-10 at a multiplicity of 10 PFU/cell and after 18 hr of incubation at 37°, the cells were labelled with 75 uCi of [³⁵S]methionine (NEN, 1154 Ci/m mole) for 2 hr in methionine-free medium. Prior to labelling, the cells were incubated for 1 hr in methionine-free medium to reduce the intracellular pools of the precursor. After the labelling periods, the medium was removed, the monolayers rinsed three times with cold PBS and the cells lysed in 500 ul of RIPA buffer (1% Triton X-100, 1% sodium deoxycholate, 0.15 M NaCl, 0.05 M Tris-HCl, 0.01 M EDTA, 0.1% SDS, pH 7.4). Aliquots of 10 ul of the extracts were incubated with 5-10 ul of the appropriate antiserum for 1 hr at room temperature before the addition of 25 µl of a suspension of 100 mg 1 ml of protein A-Sepharose CL-4B beads (Sigma, St. Louis, MO) in RIPA buffer. Following a further 1 hr incubation at room temperature, the beads were recovered by centrifugation, washed 3 times with cold RIPA buffer and the immune complexes that were bound to the beads were removed by boiling for 5 min in dissociation buffer (2.3% SDS, 10% glycerol, 5% 2-mercaptoethanol, 62.5 mM Tris-HCl, 0.01% bromophenol blue, pH 6.8) followed by centrifugation. Aliquots of the supernatant fluids were subjected to electrophoresis in 10% discontinuous gels of polyacrylamide as described by Laemmli (1970). After electrophoresis, the gels were impregnated with fixing solution (40% methanol, 10% acetic acid in water), dried and exposed at -70° C to X-ray film.

F. Production of antibodies in mice and rabbits.

S. frugiperda cells infected with the RP6S3.11 recombinant were collected and subjected to freezing and thawing, followed by low speed centrifugation. The supernatant fluids were used to raise antibodies in mice or rabbits. For mice, each animal received one intraperitoneal injection of antigen in Freund's complete adjuvant on day 0, followed by three injections of antigen in Freund's incomplete adjuvant on days 7, 14 and 21 and one intraperitoneal injection of 2 × 10⁶ Ehrlich's ascites cells on day 18. Ascitic fluids were removed at intervals from day 22 to day 34. For rabbits, each animal received one intramuscular injection of antidgen in Freund's incomplete adjuvant on days 7 and 16. Blood was collected on day 14 and on day 22.

### G. Immunoprecipitation of proteins using mouse ascitic fluids or rabbit antisera.

The mouse ascitic fluids and rabbit sera were diluted 25 time with RIPA buffer and incubated with [$^{35}$S] methionine labelled BTV-10 infected BHK-21 cell lysates for 1 hr prior to immunoprecipitation and gel analyses as described above.

### H. Indirect ELISA.

A solid-phase indirect micro-ELISA using rigid flat-bottomed polystyrene ELISA plates (NUNC, Immunoplate 1) was used for examination of recombinant virus RP6S3.11 antigen reactivity with polyclonal BTV antisera. S. frugiperda cells infected with the RP6S3.11 recombinant were collected and subjected to freezing and thawing followed by low speed centrifugation. The supernatant was diluted (1:10 to 1:10.000) with carbonate buffer (15 mM Na$^2$CO$^3$, 33 mM NaHCO$^3$, pH 9.6) and 100 of each diluted preparation was added to each well of an ELISA plate and left overnight at 4°C. The plates were washed three times between each step by flooding the wells of the plate with PBST buffer (0.0025% of Tween 20 in PBS). The BTV antisera was diluted (1:000) in PBST containing egg albumin (0.2% w/v final concentration) and polyvenylpyrollidone, m.w. 40,000 (2% w/v final concentration). The diluted antisera (100 µl in each well) were added to the absorbed antigen and incubated at 37°C for one hour. After washing the plate, 100 µl of 1:100 dilution of alkaline phosphatase conjugated protein A (Sigma Chemicals) were added to each well followed by 2 hr incubation at 28°C. The substrate, p-nitrophenylphosphate (Sigma Chemicals) solution (0.1% w/v final concentration) was added, for 30 min or less at room temperature. After suitable colour development the reaction was stopped by the addition of 75 ul of 3M NaoH. The optical density was read using a multichannel spectrophotometer (Titertek Multiskan, Flow Laboratories) at a wavelength of 405 nm.

The results achieved in the above Examples in apparent from the following discussion.

### 1.1 Construction of the recombinant viruses (RP6S2.1, RP6S2.2)

The strategy employed for the construction of the baculovirus transfer vector containing the entire BTV-10 VP2 gene is shown in Figure 1. The procedure involved the preparation of a complete DNA copy of the VP2 gene from overlapping DNA clones, removal of the terminal dC-dG sequences and insertion of the DNA into a baculovirus transfer vector as described in Example 1. The orientation and sequence of the VP2 gene in the transfer vector relative to the AcNPV polyhedrin leader were determined by DNA sequence analysis (Fig. 2). For the recombinant transfer vector pAsSI10.2 the VP2 DNA insert contained three G nucleotides (representing the residues of the dC-dG tails) followed by the entire VP2 sequence (Fig. 2, Purdy et al., 1985). In order to transfer the VP2 gene into the AcNPV genome, S. frugiperda cells were co-transfected with pAc6SI10.2 DNA and the infectious AcNPV DNA. Putative recombinant viruses were isolate from the infected cells by selecting progeny viruses with a polyhedrin-negative plaque phenotype and after 3 successive cycles of plaque purification, stocks of 2 recombinant viruses (RP6S2.1, RP6S2.2) were obtained.

### 1.2 Analysis of the recombinant viral DNA by restriction enzyme cleavage and Southern blot hybridization

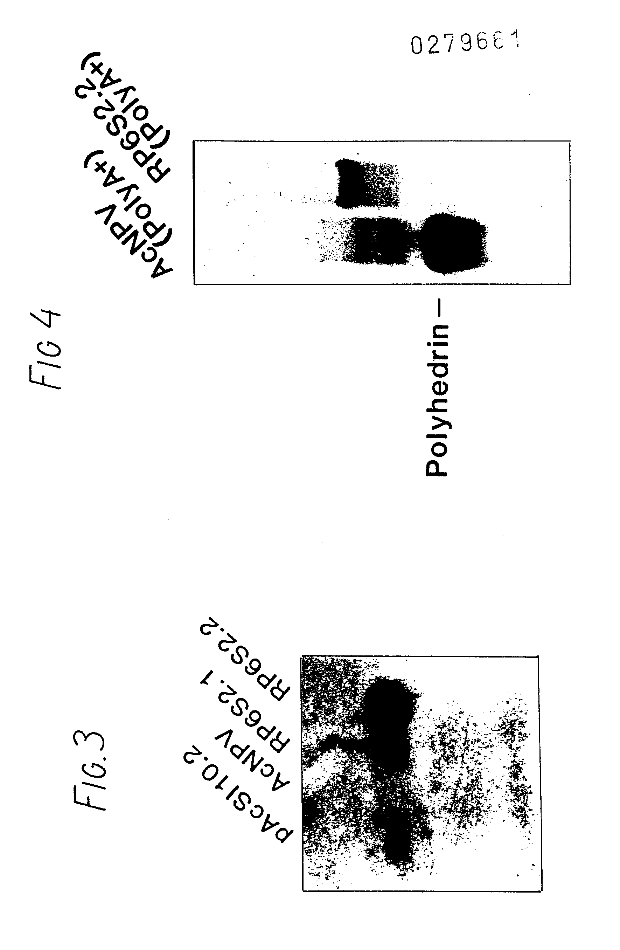

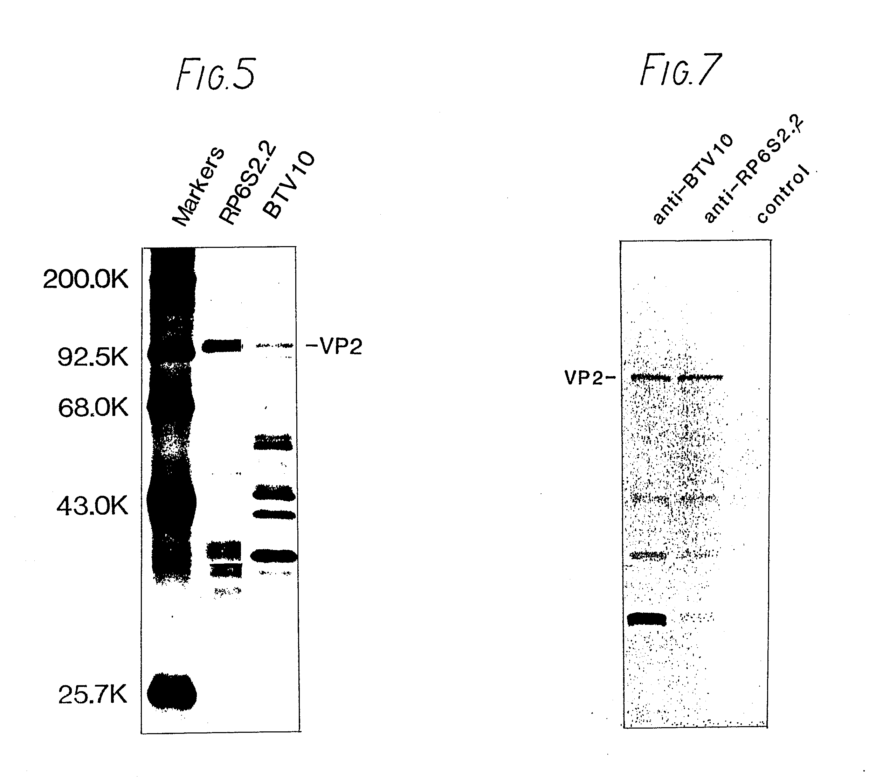

Recombinant baculoviruses were propagated, purified, and the DNA was extracted as described (Matsuura et al., 1986). The viral DNA was cleaved with the restriction endonuclease BamHI and the fragments separated by electrophoreses in 0.8% agarose gel in order to identify the fragment representing the VP2 gene. The DNA fragments were transferred to "Genescreen Plus" membrane (Southern, 1975) and the L2 gene identified by hybridization to nick-translated VP2 DNA excised from pAcSI10.2. As shown in Figure 3, both RP6S2.1 and RP6S2.2 recombinant virus DNA contained sequence which hybridized with the L2 gene. As expected, wild type AcNPV DNA did not hybridize to the probe (Fig. 3).

### 1.3 Expression of BTV-10 VP2 in S. frugiperda cells

Infection of S. frugiperda cells with the recombinant viruses did not produce nuclear inclusions. The presence of RNA species representing the BTV gene in cells infected with the RP6S2.2 recombinant virus was determined by "Northern" blot analyses. S. frugiperda cells were infected either with the RP6S2.2 recombinant virus or with wild-type AcNPV. Nucleic acids were extracted 36 hr post-infection and chromatographed on columns of oligodeoxythymidylic acid-cellulose to select RNA species with polyadenylic acid sequences. The poly (A)+ RNA preparations were treated with methylmercury hydroxide and separated into size classes by agarose gel electrophoresis in the presence of methylmercury hydroxide. RNA was blotted to "Genescreen-Plus", then hybridized with $^{32}$p-labelled nick-translated VP2 DNA to identify BTV related RNA species. As shown in Figure 4, polyadenylated RNA recovered from cells infected with the recombinant virus was identified that hybridized to the BTV probe and was estimated to be approximately 4 kb in size (i.e. corresponding to the 3 kb BTV gene plus 1 kb of the AcNPV polyhedrin gene sequence). An RNA band of 1200 bp was identified in the AcNPV virus infected cells when a 545 bp probe representing the AcNPV polyhedrin was employed. It was concluded therefore that BTV related RNA species were synthesized in the recombinant virus infected cells.

In order to determine if BTV-10 VP2 was synthesized in the recombinant virus infected cells, cells were labelled at 36 hr post-infection and the labelled proteins immunoprecipitated with the BTV-10 antisera prior to

**0 279 661**

resolution by SDS-polyacrylamide gel electrophoresis. The results of immunoprecipitation are shown in Figure 5. Control lanes in the gel included immunoprecipitates of BTV-10 infected BHK cells and protein size markers. The BTV-10 antisera precipitated a protein of approximately $93 \times 10^3$ Da from both BTV-10 infected BHK cell extracts and from the recombinant virus infected S. frugiperda cell lysates. No similar band was identified in uninfected BHK cell extracts, or in AcNPV infected or mock-infected S. frugiperda cells. These results indicated that BTV-10 VP2 protein was expressed during the recombinant virus infection.

### 1.4 Induction of BTV neutralizing antibody of a recombinant baculovirus containing the VP2 gene

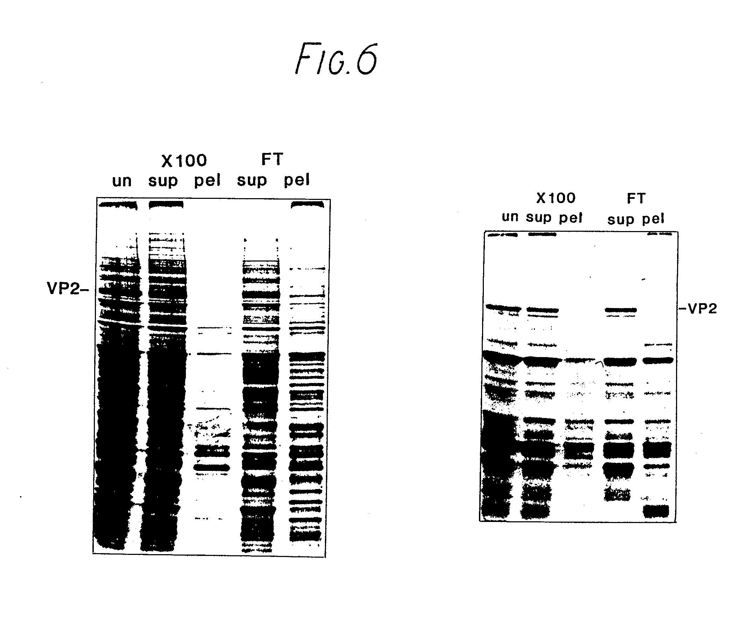

To assess the ability of the VP2 produced by the recombinant baculovirus to induce neutralizing antibodies, mice were immunized with recombinant virus infected S. frugiperda cell extracts. In preliminary experiments infected cells labelled in the presence of [$^{35}$S]methionine at 48 hr post-infection were degraded by freeze-thawing three times or by treatment with 1% Triton X100 and the extracts separated into the soluble or pellet materials by centrifugation in an Eppendorf microfuge. Aliquots of the unfractionated material or the supernatant or pelleted materials were analysed by gel electrophoresis and the proteins stained (Fig. 6, left panel) or identified by radioautography (Fig. 6, right panel). By both extraction procedures VP2 protein was identified in the supernatant materials. The supernatant fluids from the freeze-thaw extraction were used to raise antibodies in mice and rabbits as described in the Methods. Mouse ascitic fluids were collected between 22 to 34 days post immunization and were tested for antibody that would neutralize BTV-10 by the plaque reduction test. Similarly rabbit antisera were assayed for BTV-10 neutralizing antibody using samples collected 14 to 22 days post-immunization. As shown in the Table 1 the immunized mice and rabbits both produced BTV-10 neutralizing antibody with high titres. By comparison, animals inoculated with extracts of wild-type AcNPV infected S. frugiperda, or with saline, demonstrated no detectable neutralizing antibody titer to BTV-10. Both mouse ascitic fluids and rabbit sera were also tested for their ability to neutralize three other BTV serotypes, namely, BTV-11, BTV-13 and BTV-17. Both BTV-11 and BTV-17 reacted slightly with the samples (50% plaque reduction at dilutions at or less than 1:10). BTV-13 was not neutralized (Table 1).

### 1.5 Immunoprecipitation of BTV-10 VP2 by immunized mouse ascitic fluids

The mouse ascitic fluids were used in immunoprecipitation tests in order to determine whether they contained antibodies which could react with the VP2 antigen present in BTV-10 infected BHK-21 cells (Fig. 7). Immunoprecipitation studies of viral polypeptides using BTV-10 infected BHK-21 cell extracts were performed with either immunized (Fig. 7, anti-RP6S.2.2) or non-immunized mouse ascitic fluids (Fig. 7, control) and the precipitated proteins compared to the proteins precipitated with polyclonal BTV-10 antisera (Fig. 7, anti-BTV 10). The immunized mouse ascitic fluids, but not the control material, precipitated VP2 from the BHK-21 infected cell extracts. The observation that some other viral proteins were also recovered may indicate that in infected cell extracts VP2 is associated with other structural proteins of the virus. Similar results were obtained using the rabbit antisera.

### 2.1 Construction of the recombinant virus (RP6S3.11).

The strategy for the construction of the baculovirus transfer vector containing the entire VP3 gene is shown in Fig. 8, The procedure involved the removal of the terminal dA-dT sequences and insertion of the DNA into a baculovirus transfer vector as described in Methods. The orientation and sequence of the L3 gene in the transfer vector relative to the AcNPV polyhedrin leader were determined by DNA sequence analysis (Fig. 9). For the recombinant transfer vector pAcSI17.3 the VP3 DNA insert contained one T nucleotide (representing the residues of the dA-dT tails) followed by the entire L3 sequence (Fig. 3, Purdy et al., 1984). In order to transfer the L3 gene into the AcNPV genome, S. frugiperda cells were co-transfected with pACSI17.3 DNA and the infectious viruses were isolated from the infected cells by selecting progeny viruses with a polyhedrin-negative plaque phenotype and after 3 successive cycles of plaque purification, stocks of recombinant virus (RP6S3.11) were obtained.

### 2.2 Analysis of the recombinant viral DNA by restriction enzyme cleavage and Southern blot hybridization.

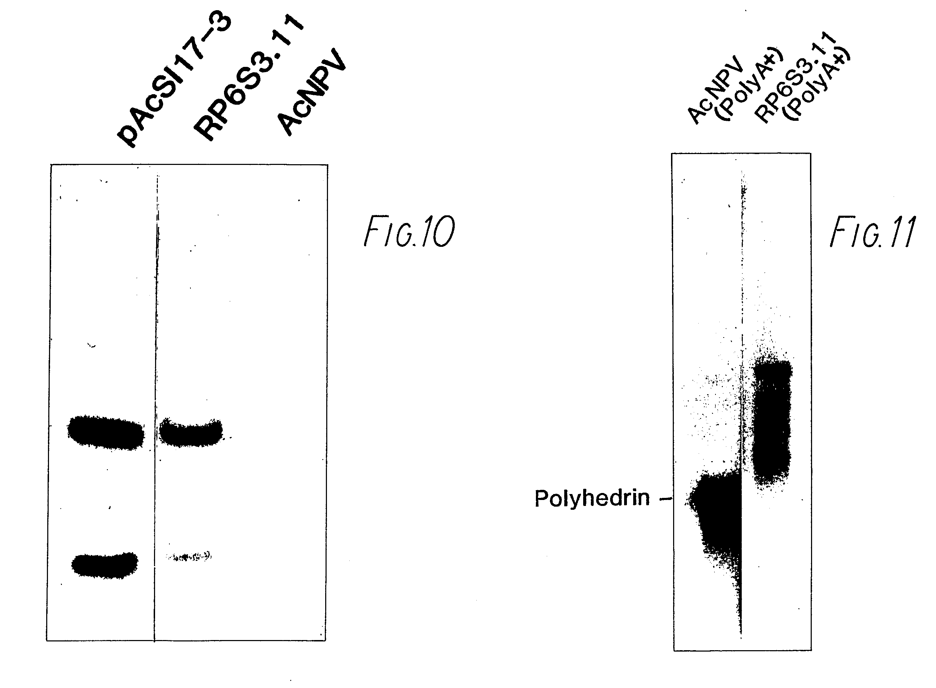

Recombinant virus, RP6S3.11 was propagated, purified, and the DNA was extracted as described (Matsuura et al., 1986). The viral DNA was cleaved with the restriction endonuclease (EcoRV and HindIII and the fragments separated by electrophoresis in 0.8% agarose gel in order to identify the fragments representing the L3 gene. The DNA fragments were transferred to "Genescreen Plus" membrane (Southern, 1975) and the L3 gene identified by hybridization to nick-translated L3 DNA excised from pAcSI17.3. As shown in Fig. 3, RP6S3.11 recombinant virus DNA contained sequence which hybridized with the L3 gene. Since segment 3 DNA has one internal EcoRV site (Purdy et al., 1984), two hybridized bands were visualized in both RP6S3.11 and pACSI.17.3 lanes. As expected AcNPV DNA did not hybridize to the probe (Fig. 10).

### 2.3 Expression of BTV-17 VP3 in S. frugiperda cells.

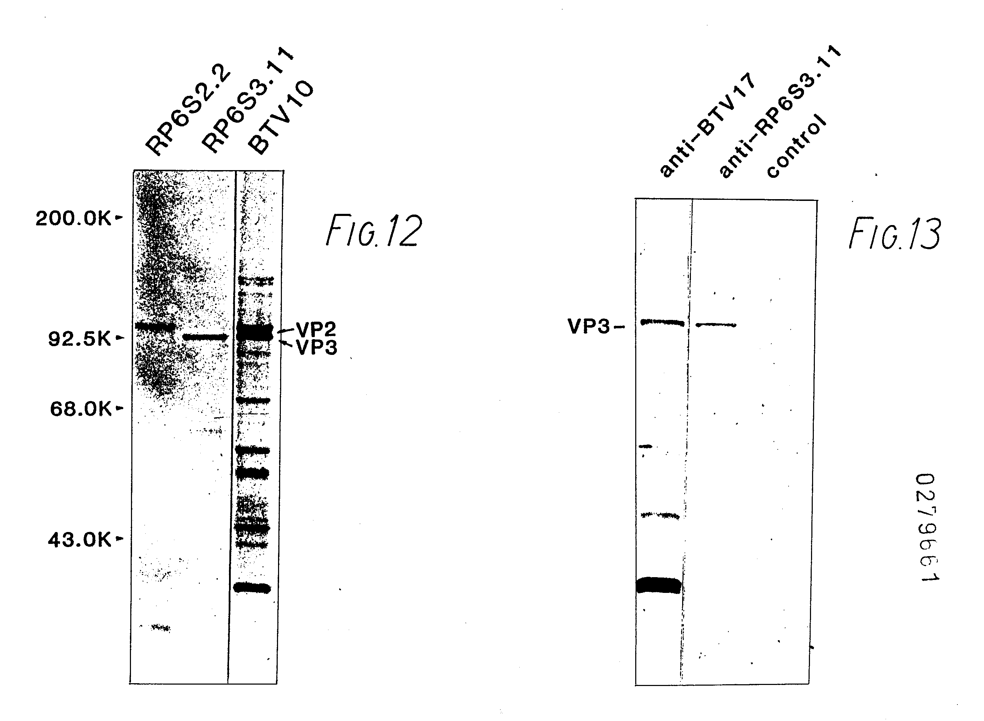

Infection of S. frugiperda cells with the recombinant viruses did not produce nuclear inclusions. The presence of RNA species representing the BTV gene in cells infected with the RP6S3.11 recombinant virus was determined by "Northern blot" analyses. S. frugiperda cells were infected either with the RP6S3.11 recombinant virus or with wild-type AcNPV. Nucleic acids were extracted 36 hr post-infection and chromatographed on columns of oligodeoxythymidylic acid-cellulose to select RNA species with polyadenylic

7

acid sequences. The poly (A) + RNA preparations were treated with methylmercury hydroxide and separated into size classes by agarose gel electrophoresis in the presence of methylmercury hydroxide. RNA was blotted to "Genescreen-Plus", then hybridized with $^{32}$P-labelled nick-translated L3 DNA to identify BTV related RNA species. As shown in Fig. 11, polyadenylated RNA recovered from cells infected with the recombinant virus was identified that hybridized to the BTV probe and was estimated to be approximately 4 kb in size (i.e corresponding to the 2.8 kb BTV gene plus 1.2 kb of the AcNPV polyhedrin gene sequence). An RNA band of 1200 bp was identified in the AcNPV virus infected cells when a 545 bp probe representing the AcNPV polyhedrin was employed (Howard et al., 1986). It was concluded, therefore, that BTV related RNA species were synthesized in the recombinant virus infected cells.

In order to determine if BTV-17 VP3 was synthesized in the recombinant virus infected cells, cells were labelled at 36 hr post-infection and the labelled proteins immunoprecipitated with the BTV-17 or BTV-10 antisera prior to resolution by SDS-polyacrylamide gel electrophoresis. The results of immunoprecipitation are shown in Fig. 12. Control lanes in the gel included immunoprecipitates of BTV-10 infected BHK cells, immunoprecipitates of cell lysates with a recombinant virus RP6S2.2 containing VP2 protein (approximately 93 × 10[3] daltons, Inumaru & Roy, 1986), and protein size markers (not shown). The BTV-10 antisera precipitated a protein of approximately 92.5 × 10[3] Da from both BTV-10 infected BHK cell extracts and recombinant virus infected S. frugiperda cell lysates. No similar band was identified in uninfected BHK cell extracts, or in AcNPV infected or mock-infected S. frugiperda cells. These results indicated that BTV-17 VP3 protein was expressed during the recombinant virus infection.

2.4 Immunoprecipitation of BTV VP3 by immunized mouse ascitic fluids.

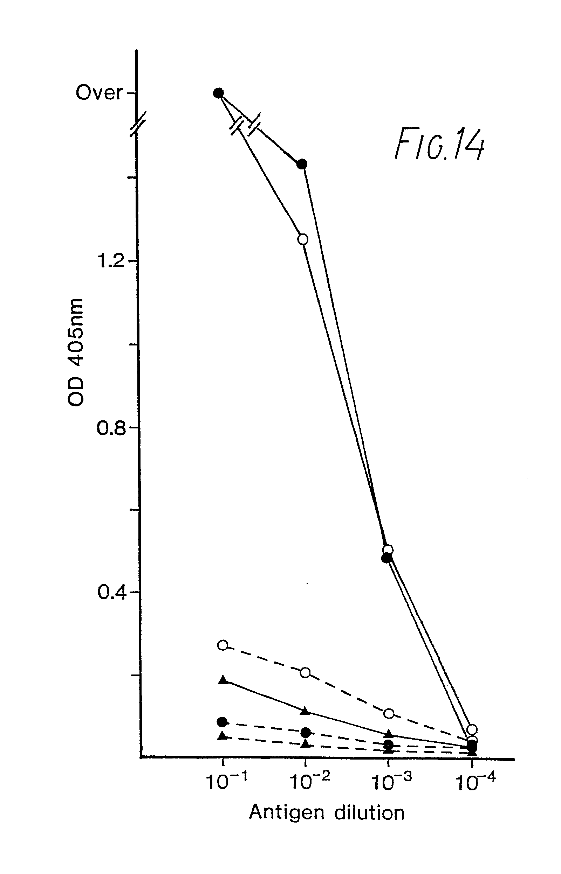

To assess the ability of VP3, produced by the recombinant baculovirus, to induce antibodies which could react with VP3 antigen present in BTV infected BHK-21 cells, mice were immunized with recombinant virus infected S. frugiperda cell extracts as described in Methods. Ascitic fluids were collected between 22 and 34 days post immunization and were used in immunoprecipitation tests in order to determine whether they contained antibodies which could react with the VP3 antigen present in BTV-10 or BTV-17 infected BHK-21 cells. Immunoprecipitation studies of viral polypeptides using BTV-10 or BTV-17 infected BHK-21 cell extracts were performed with either immunized (Fig. 13, anti-RP6S3.11) or non-immunized mouse ascitic fluids (Fig. 13, control) and the precipitated proteins compared to the proteins precipitated with polyclonal BTV-17 antisera (Fig. 13, anti-BTV-17). The immunized mouse ascitic fluids, but not the control material, precipitated VP3 from both BTV-10 and BTV-17 infected BHK-21 cell extracts.

2.5 Reactivity of the baculovirus expressed VP3 antigen with BTV antisera using an ELISA test.

In order to determine if VP3 protein produced by the recombinant baculovirus could react with homologous or heterologous BTV antisera, the recombinant virus infected S. frugiperda cell extracts were adsorbed to the microtiter plates as described in the Methods. The adsorbed antigen was then incubated either with BTV-17 or BTV-10, antisera or with non-immune sera as described in Methods, As a control, AcNPV infected cells were also adsorbed and incubated with each of the sera. The antigen-antibody complexes were than detected by incubating each with alkaline phosphatase protein-A conjugate followed by the addition of a substrate as described in Methods. As shown in Fig. 14, both BTV antisera (1:1000 dilution) reacted with recombinant antigen in a significantly high level, while reaction with AcNPV antigen was almost negligible. The normal rabbit serum did not react either with recombinant antigen or with wild type AcNPV antigen. When BTV-11 or BTV-13 antisera were tested in similar experiments, both antisera also reacted positively.

It can be seen from the above results that Baculovirus recombinants between AcNPV DNA and the bluetongue virus type 10 segment 2 gene which codes for the neutralization antigen VP2 have been constructed and used to synthesize the VP2 antigen in insect cell cultures. The BTV-10 VP2 protein that was produced in the insect expression system reacted with BTV-10 to elicit antibodies in mice and rabbits. The antibodies that were produced recognized VP2 protein induced in BTV-10 infected cells and neutralized the infectivity of BTV-10 virus and to lesser extent certain other BTV serotypes. Thus, in its biological activities the recombinant virus-produced VP2 resembles authentic bluetongue virus VP2 polypeptide. Our experiments have raised the possibility that the protein expressed by the baculovirus recombinant may be of value as a subunit vaccine for BTV.

It can further be seen that recombinant baculoviruses containing segment 3 gene of bluetongue virus type 17 have been constructed and used to express the group-specific antigen of bluetongue in insect cells of S. frugiperda. Immunoprecipitation of the VP3 protein produced in insect cells showed that the product was similar to the bluetongue virus VP3 both in antigenicity and in size. The expressed VP3 antigen was also shown to elicit antibodies in mice and rabbits that recognize BTV protein. Furthermore, the recombinant virus extract was demonstrated to be reactive with at least four bluetongue antisera (so far tested) in an indirect ELISA test.

The expression of antigenically-active polypeptides derived from bluetongue virus in accordance with the invention is a particularly surprising and valuable result. First it is noteworthy that an expression system based on a recombinant baculovirus is capable of expressing bluetongue virus proteins, particularly having regard to the lack of success of other workers using different expression systems. Secondly expression of bluetongue proteins in a non-ungulate expression system in antigenically active form is a major achievement. Further, native bluetongue antigens comprise large structurally complex entities and it could not have been foreseen that polypeptides produced by recombinant DNA techniques (especially using an insect, e.g. baculovirus, expression system) would have appropriate conformations, i.e. conformations corresponding to those of native bluetongue proteins.

The value, both scientifically and commercially, of the invention is that the polypeptides produced have a high potential for use as or in the manufacture of vaccines and diagnostic reagents. This particularly applies to VP3 which a group-specific. This genetically engineered bluetongue antigen is thus of particular utility as a reagent for the diagnosis of bluetongue.

Recombinant baculoviruses described herein have been deposited with the American Type Culture Collection and assigned the following deposit numbers:-

Recombinant BTV-10 Segment 2    ATCC VR2165
Baculovirus RP6S.2    15th April 1987

Recombinant BTV-17 Segment 3    ATCC VR2166
Baculovirus RP6S3.11    15th April 1987

TABLE 1

Neutralization titers of antibodies raised in mice or a rabbit to

extracts of S. frugiperda cells infected with recombinant

baculoviruses expressing the BTV-10 VP2 gene

|  | Antibody titers to: | | | |
|---|---|---|---|---|
| Antiserum | BTV-10 | BTV-11 | BTV-13 | BTV-17 |
| Mouse ascitic fluid | 640 | 40 | 0 | >10 |
| Rabbit antisera | 640 | 40 | 0 | >10 |
| Preimmune sera | 0 | 0 | 0 | 0 |

Neutralization titers were determined by plaque reduction assays and are expressed as the reciprocal of serum/ascitic fluid dilution that reduced plaque numbers of 50%. Preimmune sera from each animal were included as controls.

**Claims**

1. A process for producing a polypeptide comprising at least an antigenic portion of a bluetongue virus protein which comprises infecting susceptible insects or cultured insect cells with an expression vector having an DNA segment coding for said polypeptide.

2. A process according to Claim 1 wherein said DNA segment codes for at least an antigenic portion of a core protein of a BTV particle.

3. A process according to Claim 1 wherein said DNA segment codes for at least an antigenic portion of a surface protein of a BTV particle.

4. A process according to Claim 1 wherein said DNA segment codes for at least an antigenic portion of bluetongue VP2 protein.

5. A process according to Claim 1 wherein said DNA segment codes for at least an antigenic portion of bluetongue VP3 protein.

6. A process according to any preceding claim wherein the expression vector comprises a recombinant baculovirus.

7. A recombinant baculovirus suitable for use in the process of any of Claims 1 to 6 comprising a DNA segment coding for polypeptide comprising at least an antigenic portion of a bluetongue virus protein and a promoter capable of directing expression of said polypeptide in an infected insect or in infected insect cells.

8. A polypeptide produced by the process of any of Claims 1 to 6.

9. A vaccine comprising a polypeptide as claimed in Claim 8.

0279661

FIG.1

# FIG. 2

|  | BamH I | Sma I | BamH I |
|---|---|---|---|

pAcRP6S     —GTAATAAAAAAACCCCGGATCCCCGGGGATCCTTTCCT—

pAcSI10.2    —GTAATAAAAAAACCCCGGATCCCCGGGGTTAAAAGAGTGTTCTACCATGGAGGAA —
                tail   → BTV10 segment 2

FIG.3

FIG 4

Polyhedrin —

## FIG.5

Markers    RP6S2.2    BTV10

200.0K

92.5K — — −VP2

68.0K

43.0K

25.7K

## FIG.7

anti-BTV10    anti-RP6S2.?    control

VP2−

0279661

# FIG.6

X100    FT
un sup pel sup pel

VP2—

X100    FT
un sup pel   sup pel

—VP2

0279661

FIG.8

# FIG. 9

BamH I

pAcRP6  —GTAATAAAAAAACCCCGGATCCTTTCCT—

pAcSI17.3  —GTAATAAAAAAACCCCGGATCGGGTGTTAAATTTCCGTAGCCATGGCTGCT—

half
Sma I  tail  → BTV17 segment 3

0279661

Fig.11

Fig.10

ACNPV (polyA+)

RP6S3.11 (polyA+)

Polyhedrin

pACSI17-3

RP6S3.11

ACNPV

FIG.12

FIG.13

RP6S2.2
RP6S3.11
BTV10

200.0K ▸

92.5K ▸    ←VP2
           ←VP3

68.0K ▸

43.0K ▸

anti-BTV17
anti-RP6S3.11
control

VP3 —

0279661

0279661

FIG.14

OD 405nm

Antigen dilution